(19) Europäisches Patentamt
European Patent Office
Office européen des brevets

(11) Veröffentlichungsnummer: **0 170 987**
**A1**

# (12) EUROPÄISCHE PATENTANMELDUNG

(21) Anmeldenummer: **85109288.2**

(22) Anmeldetag: **24.07.85**

(51) Int. Cl.⁴: **C 07 D 235/24**, C 07 D 401/12, C 07 D 403/12, C 09 B 29/036

(30) Priorität: **02.08.84 DE 3428493**

(43) Veröffentlichungstag der Anmeldung: **12.02.86**
**Patentblatt 86/7**

(84) Benannte Vertragsstaaten: **CH DE FR GB IT LI**

(71) Anmelder: **BAYER AG, Konzernverwaltung RP**
**Patentabteilung, D-5090 Leverkusen 1 Bayerwerk (DE)**

(72) Erfinder: **Bornatsch, Wolfgang, Dr., Baldurstrasse 21, D-5000 Köln 91 (DE)**
Erfinder: **Schündehütte, Karl Heinz, Prof. Dr., Klief 75, D-5090 Leverkusen 3 (DE)**

## (54) Benzimidazol-2-carbonsäure-Derivate.

(57) Benzimidazol-2-carbonsäure-Derivate die in einer ihrer tautomeren Formen der Formel

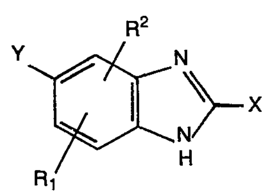

I

entsprechen, in der

$R_1$, $R_2$ H, Halogen, -R', -OR', -SR', -OH, 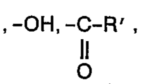 ,

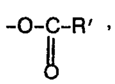 , 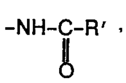 , 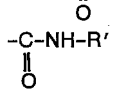 ,

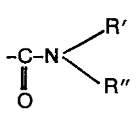 , 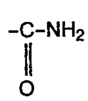 $-SO_2-NH_2$, $-SO_2-NH-R'$,

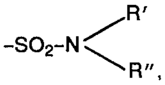 , $-NH-SO_2R'$, -CN, 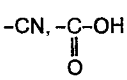 und $-NO_2$

bezeichnen, wobei
R', R'' für gegebenenfalls substituiertes Alkyl, gegebenenfalls substituiertes Cycloalkyl, gegebenenfalls substituiertes Aryl und gegebenenfalls substituiertes Aralkyl stehen,
X eine Carboxylfunktion oder derivatisierte Carboxylfunktion bezeichnet und
Y für einen Rest der Formeln

a) $-NH_2$

b) 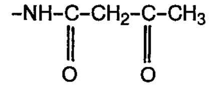

c) 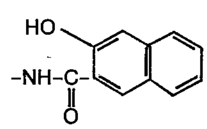

d) 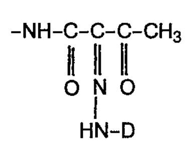

ACTORUM AG

e) 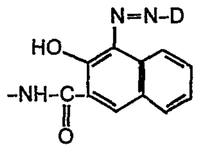

f) -N=N-K steht,

wobei
D den Rest einer Diazokomponente und
K den Rest einer Kupplungskomponente bezeichnen,
sowie die Verwendung der Benzimidazol-2-carbonsäurederivate als Farbstoffe und Farbstoffvorprodukte.

BAYER AKTIENGESELLSCHAFT 5090 Leverkusen, Bayerwerk
Konzernverwaltung RP
Patentabteilung PG/Em

## Benzimidazol-2-carbonsäure-Derivate

Die Erfindung betrifft Benzimidazol-2-carbonsäure-Derivate die in einer ihrer tautomeren Formen der Formel

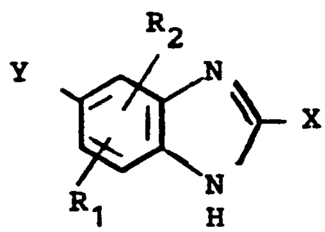

(I)

entsprechen, in der

$R_1$, $R_2$ H, Halogen, $-R'$, $-OR'$, $-SR'$, $-OH$, $-\underset{\overset{\|}{O}}{C}-R'$,

$-O-\underset{\overset{\|}{O}}{C}-R'$, $-NH-\underset{\overset{\|}{O}}{C}-R'$, $-\underset{\overset{\|}{O}}{C}-NH-R'$, $-\underset{\overset{\|}{O}}{C}-N\begin{matrix}R'\\R''\end{matrix}$, $-\underset{\overset{\|}{O}}{C}-NH_2$,

$-SO_2-NH_2$, $-SO_2-NH-R'$, $-SO_2-N\begin{matrix}R'\\R''\end{matrix}$, $-NH-SO_2R'$, $-CN$, $-\underset{\overset{\|}{O}}{C}-OH$, und $-NO_2$ bezeichnen,

wobei

R', R" für gegebenenfalls substituiertes Alkyl, gegebenenfalls substituiertes Cycloalkyl, gegebenenfalls substituiertes Aryl und gegebenenfalls substituiertes Aralkyl stehen,

X eine Carboxylfunktion oder derivatisierte Carboxylfunktion bezeichnet und

Y für einen Rest der Formeln

a) $-NH_2$

b) 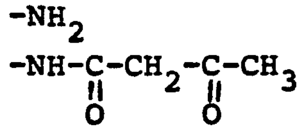

c) 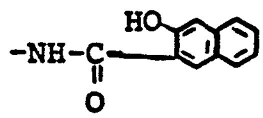

d) 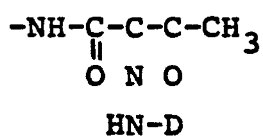

e) 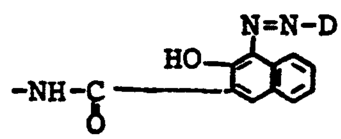

f) -N=N-K steht,

wobei

D den Rest einer Diazokomponente und

K den Rest einer Kupplungskomponente bezeichnen.

Halogen ($R_1$, $R_2$) bezeichnet bevorzugt Cl, F und Br. Gegebenenfalls substituiertes Alkyl (R', R") bezeichnet vorzugsweise $C_1$-$C_8$-Alkyl, insbesondere $C_1$-$C_4$-Alkyl, z.B. Methyl, Ethyl, n-Propyl, i-Propyl, n-, i- und t-Butyl, wobei die Alkylreste beispielsweise durch $C_1$-$C_4$-Alkoxy wie Methoxy, Ethoxy, n- und i-Propyloxy und n-, i- und t-Butyloxy, $C_1$-$C_4$-Alkylthio wie Methylthio, Ethylthio, n- und t-Butylthio, Halogen wie Chlor, Brom und Fluor, Hydroxy, Cyan, Nitro, Amino, Mono- und Di-$C_1$-$C_4$-alkylamino wie Methylamino, Methylethylamino, n- und i-Propylamino, Methyl-n-butylamino, Carboxy, $C_1$-$C_4$-Alkoxy-carbonyl wie Methoxycarbonyl und Ethoxycarbonyl, Sulfo, $C_1$-$C_4$-Alkylsulfonyl wie Methylsulfonyl und Ethylsulfonyl und Arylsulfonyl mit 6 bis 10 C-Atomen im Arylteil wie Phenylsulfonyl substituiert sein können.

Gegebenenfalls substituiertes Cycloalkyl (R',R") bezeichnet bevorzugt mono-, bi- und tricyclisches Cycloalkyl mit 3 bis 10, insbesondere 3 bis 7 C-Atomen, z.B. Cyclopropyl, Cyclobutyl, Cyclopentyl, Cyclohexyl und Cycloheptyl, wobei die Cycloalkylreste beispielsweise durch $C_1$-$C_4$-Alkoxy wie Methoxy, Ethoxy, n- und i-Propyloxy und n-, i- und t-Butyloxy, $C_1$-$C_4$-Alkylthio wie Methylthio, Ethylthio, n- und i-Propylthio und n-, i- und t-Butylthio, Halogen wie Chlor, Brom und Fluor, Hydroxy, Cyan, Nitro, Amino, Mono- und Di-$C_1$-$C_4$-alkylamino wie Methylamino Methylethylamino, n- und i-Propylamino, Methyl-n-butylamino, Carboxy, $C_1$-$C_4$-Alkoxy-carbonyl wie Methoxycarbonyl und Ethoxycarbonyl, Sulfo, $C_1$-$C_4$-Alkylsulfonyl wie Methylsulfonyl und Ethylsulfonyl und Arylsulfonyl mit 6 bis 10

C-Atomen im Arylteil wie Phenylsulfonyl substituiert sein können.

Gegebenenfalls substituiertes Aryl (R',R") bezeichnet bevorzugt Aryl mit 6 bis 10 C-Atomen im Arylteil, z.B. Phenyl und Naphthyl, wobei die Arylreste beispielsweise durch $C_1$-$C_4$-Alkyl wie Methyl, Ethyl, n- und i-Propyl, n-, i- und t-Butyl, $C_1$-$C_4$-Alkoxy wie Methoxy, Ethoxy, n- und i-Propyloxy und n-, i- und t-Butyloxy, $C_1$-$C_4$-Alkylthio wie Methylthio, Ethylthio, n- und i-Propylthio und n-, i- und t-Butylthio, Halogen wie Chlor, Brom und Fluor, Hydroxy, Cyan, Nitro, Amino, Mono- und Di-$C_1$-$C_4$-alkylamino wie Methylamino, Methylethylamino, n- und i-Propylamino, Methyl-n-butylamino, Carboxy, $C_1$-$C_4$-Alkoxycarbonyl wie Methoxycarbonyl und Ethoxycarbonyl, Sulfo, $C_1$-$C_4$-Alkylsulfonyl wie Methylsulfonyl und Ethylsulfonyl und Arylsulfonyl mit 6 bis 10 C-Atomen im Arylteil wie Phenylsulfonyl substituiert sein können.

Gegebenenfalls substituiertes Aralkyl (R',R") bezeichnet bevorzugt Aryl-$C_1$-$C_6$-alkyl, insbesondere Aryl-$C_1$-$C_4$-alkyl wie Phenyl-$C_1$-$C_4$-alkyl und Naphthyl-$C_1$-$C_4$-alkyl, z.B. Benzyl und Phenethyl, wobei die Arylreste beispielsweise durch $C_1$-$C_4$-Alkyl wie Methyl, Ethyl, n- und i-Propyl, n-, i- und t-Butyl, $C_1$-$C_4$-Alkoxy wie Methoxy, Ethoxy, n- und i-Propyloxy und n-, i- und t-Butyloxy, $C_1$-$C_4$-Alkylthio wie Methylthio, Ethylthio, n- und i-Propylthio und n-, i- und t-Butylthio, Halogen wie Chlor, Brom und Fluor Hydroxy, Cyan, Nitro, Amino, Mono- und Di-$C_1$-$C_4$-alkylamino wie Methylamino, Methylethylamino, n- und i-Propylamino, Methyl-n-butylamino, Carboxy, $C_1$-$C_4$-Alkoxy-carbonyl wie Methoxycarbonyl und Ethoxycarbonyl, Sulfo, $C_1$-$C_4$-Alkylsulfonyl wie Methylsulfonyl und Ethylsulfonyl und Arylsulfonyl mit 6 bis 10 C-Atomen im Arylteil wie Phenylsulfonyl substituiert sein können.

$R_1$ und $R_2$ stehen bevorzugt für Wasserstoff.

X bezeichnet bevorzugt -COOH, -COOR', $-CONH_2$, -CONHR',
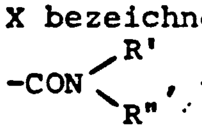, -CN.

Besonders bevorzugt steht X für -COOH, $-COOCH_3$, $-COOC_2H_5$, $-CONH_2$, $-CONHCH_3$, $-CONHC_2H_5$, $-CON(CH_3)_2$, $-CON(C_2H_5)_2$ und -CONH-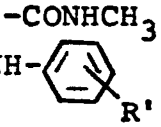.

Von ganz besonderer Bedeutung sind Verbindungen der Formel I, bei denen X $-CONH_2$ und $-CONHCH_3$ bezeichnet.

Bei D handelt es sich bevorzugt um den Rest einer Diazokomponente, die so ausgewählt wird, daß das Gesamtmolekül Id oder Ie Pigmenteigenschaften besitzt.

Insbesondere ist D der Rest einer Diazokomponente aus der Benzol-, Naphthalin-, Anthrachinon- oder heterocyclischen Reihe wie Phenyl, Naphthyl, oder Anthrachinonyl, die vorzugsweise 1,2 oder 3 gleiche oder verschiedene Substituenten $R_1$ in der zu Formel I angegebenen Bedeutung tragen können, ein gegebenenfalls substituierter Barbitursäurerest z.B. ein Cyaniminobarbitursäurerest oder

ein Rest, der in einer seiner tautomeren Formen der Formel

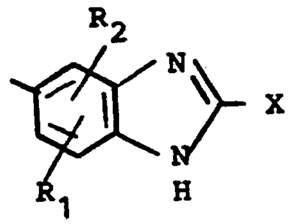

II

entspricht,

wobei $R_1$, $R_2$ und X die zu Formel I angegebenen Bedeutungen gaben.

Besonders bevorzugt ist D ein Phenylrest, der durch Nitro, $C_1$-$C_4$-Alkoxy, insbesondere Methoxy, Carboxy, Trifluormethyl, Halogen, insbesondere F, Cl, Br, $C_1$-$C_4$-Alkyl, Carbamoyl oder einen anderen, gegebenenfalls in gleicher Weise substituierten Phenylrest, substituiert sein kann.

Weiterhin steht D bevorzugt für einen Rest der Formel II, bei dem $R_1$, $R_2$ für Wasserstoff und X für $-CONH_2$ oder $-CONHCH_3$ stehen.

Bei K handelt es sich bevorzugt um eine Kupplungskomponente aus der Benzol-, Naphthalin-, Acetessigamid- oder heterocyclischen Reihe, z.B. ein Rest, der sich von einem 2-Hydroxy-3-naphthoesäurearylid, einem Acetessigarylid, einer gegebenenfalls substituierten Barbitursäure wie Cyaniminobarbitursäure, einem Pyrazolon oder einem 3-Cyan-4-methyl-2,6-pyridon ableitet, wobei unter Arylid vorzugsweise ein Phenylrest verstanden wird, der beispielsweise durch $R_1$ in der zu Formel I angegebenen Bedeutung substituiert sein kann oder an den ein heterocyclischer

Rest ankondensiert sein kann, so daß z.B. insgesamt ein Rest der Formel II entsteht.

Besonders bevorzugt handelt es sich bei dem Arylidrest um einen Phenylrest, der 1,2 oder 3 Substituenten aus der Reihe $C_1$-$C_4$-Alkyl, insbesondere Methyl, $-CF_3$, $C_1$-$C_4$-Alkoxy, insbesondere Methoxy und Halogen, insbesondere F, Cl, Br trägt.

Bei den Kupplungskomponenten aus der Pyrazolonreihe handelt es sich bevorzugt um 5-Hydroxy-3-methyl- oder 5-Hydroxy-3-carboxypyrazole, die in 1-Stellung H oder einen gegebenenfalls substituierten Arylrest tragen, wobei der gegebenenfalls substituierte Arylrest die oben zu R', R" angegebenen Bedeutungen hat, wobei Phenyl und p-Tolyl bevorzugt sind.

Die Amine der Formel Ia können nach literaturbekannten Verfahren oder in Analogie dazu hergestellt werden; s. z.B. (1) Chem. Heterocycl. Compds. (USSR) 18, 519 (1982); (2) Chem. Abst. 65, 8894 (1966); (3) J. Amer. Chem. Soc. (C) 1750, 1967; und für die Reduktion (5) Houben-Weyl 11/1, 499, Georg Thieme Verlag Stuttgart 1957 und (6) Houben-Weyl 10/1, 1257, Georg Thieme Verlag Stuttgart 1971.

So hat sich die folgende Darstellungsweise bewährt, die aus den Literaturstellen (4), (5) und (6) bekannt ist und z.B. gemäß dem folgenden Reaktionsschema verläuft:

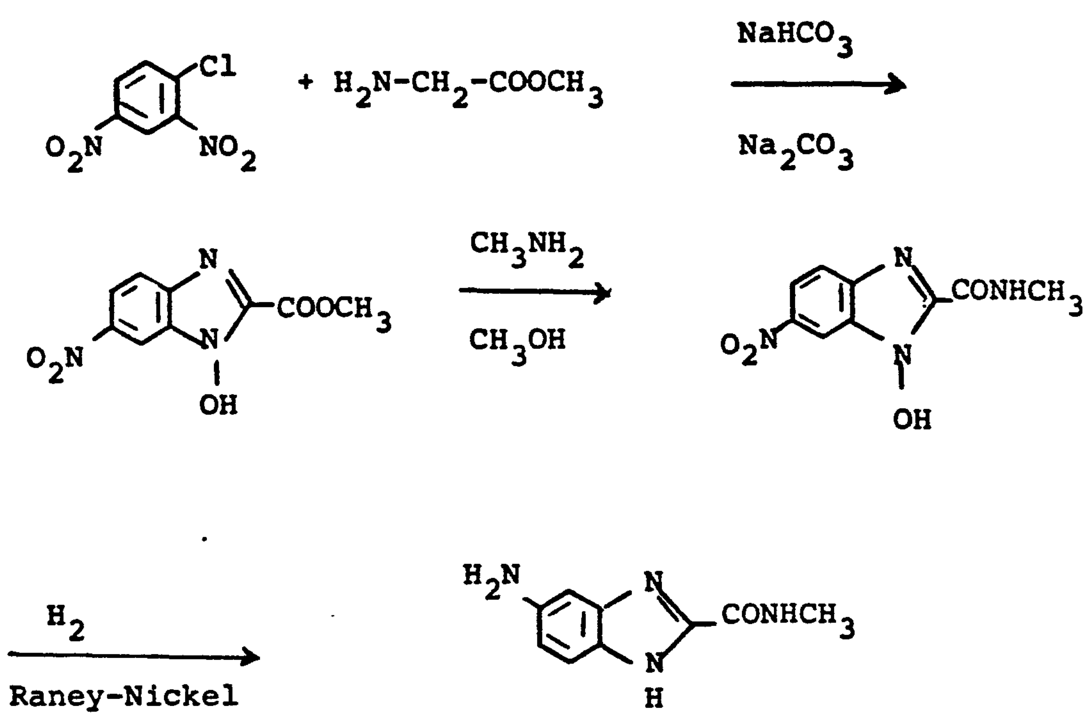


Die Acetessigarylide der Formel Ib werden z.B. dadurch erhalten, daß man die Amine der Formel Ia in Eisessig mit Diketen nach im Prinzip bekannten Verfahren umsetzt.

Die 2-Hydroxy-3-naphthoesäurearylide der Formel Ic werden ebenfalls nach im Prinzip bekannten Verfahren durch Umsetzung von 2-Hydroxy-3-naphthoesäure mit dem Amin der Formel Ia in Gegenwart eines wasserentziehenden Mittels wie Phosphortrichlorid oder Thionylchlorid eventuell in einem inerten organischen Lösungsmittel hergestellt (s. z.B. DE-PS 264 527; Friedländer 12, 910 (1917); DE-PS 293 897; Friedländer 12, 912 (1917)).

Die Verbindungen der Formeln I d und Ie werden durch

Kupplung von diazotierten Aminen der Formel $D-NH_2$ auf Verbindungen der Formeln Ib und Ic hergestellt. Zur Herstellung von Verbindungen der Formel If diazotiert man ein Amin der Formel Ia und kuppelt auf eine Kupplungskomponente der Formel K-H.

Die Kupplungsreaktionen zur Herstellung der Verbindungen Id, Ie und If können in bekannter Weise durchgeführt werden, z.B. in organischen Lösungsmitteln, insbesondere aber im wäßrigen Medium, vorteilhaft in Gegenwart nichtionogener, anionaktiver oder kationaktiver Dispergiermittel und/oder organischer Lösungsmittel.

Zur Erzielung einer besonders günstigen Kristallstruktur ist es zweckmäßig, das Kupplungsgemisch einige Zeit zu erhitzen, beispielsweise zum Kochen oder unter Druck auf Temperaturen über 100 °C zu bringen, gegebenenfalls in Gegenwart organischer Lösungsmittel, wie Dichlorbenzol oder Dimethylformamid oder von Harzseife. Besonders reine und echte Färbungen erhält man mit den erfindungsgemäßen Farbstoffen, wenn man die Farbstoffe nach der Kupplung als feuchte Preßkuchen oder als getrocknete Pulver einer Nachbehandlung mit organischen Lösungsmitteln wie Pyridin, Dimethylformamid, Ethanol, Methanol, Propanol, Butanol, Toluol, Xylol, Glykol, Glykolmonomethylether, Eisessig, Chlorbenzol, Dichlorbenzol oder Nitrobenzol bei Rückflußtemperatur oder unter Druck bei erhöhter Temperatur unterwirft oder eine Mahlung der Farbstoffe unter Zusatz von Mahlhilfsmitteln anschließt.

Zur Nachbehandlung bei erhöhter Temperatur können auch wäßrig-organische Gemische wie Butanol-Wasser in beliebigen Mischungsverhältnissen, vorzugsweise 1 : 1 bis 9 : 1 verwendet werden; auch Wasser ohne Zusatz organischer Lösungmittel kann verwendet werden.

Die Verbindungen können auch in Gegenwart von Trägersubstanzen, die zur Farblackherstellung geeignet sind, gekuppelt werden.

Bei den Verbindungen der Formeln Id, Ie und If handelt es sich um wertvolle Farbstoffe, insbesondere Pigmentfarbstoffe.

Die neuen Pigmentfarbstoffe eignen sich zur Herstellung von Druckfarben, Farblacken und Dispersions-Anstrichfarben, zum Färben von Kautschuk, Kunststoffen und natürlichen oder synthetischen Harzen. Die neuen Farbstoffe sind ferner geeignet für den Pigmentdruck auf Substraten, insbesondere Textilfasermaterialien oder anderen flächenförmigen Gebilden, wie beispielsweise Papier. Die Farbstoffe können auch für andere Anwendungszwecke, z.B. in fein verteilter Form zum Färben von Kunstseide aus Viskose oder Zelluloseethern bzw. -estern, Polyamiden, Polyurethanen, Polyglykolterephthalaten oder Polyacrylnitril in der Spinnmasse oder zum Färben von Papier verwendet werden.

Die Pigmente lassen sich in den genannten Medien gut verarbeiten.

Die Färbungen weisen gute Licht-, Wetter- und Migrationsechtheiten auf und sind gegen Hitzeeinwirkung und den Einfluß von Chemikalien z.B. Lösungsmitteln beständig.

Wie oben ausgeführt dienen die Verbindungen der Formeln Ia, Ib und Ic als Zwischenprodukte zur Herstellung der neuen Farbstoffe Id, Ie und If.

Beispiel 1

3.0 g 2,4,5-Trichloranilin werden in 12 ml 36 %ige Salzsäure 14 Std. lang angerührt. Durch Zugabe von Eis wird auf 0°C gekühlt. Es werden 3.4 ml 30 %ige $NaNO_2$-Lösung in 30 min zugetropft. Man rührt 2 Std. bei 0-5°C nach. Überschüssiges Nitrit wird dann durch Zugabe von Amidosulfonsäure zerstört.
4.36 g der Verbindung

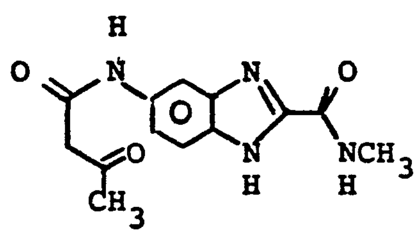

werden in 20 ml Wasser mit 3.5 ml 40 %iger Natronlauge bei Raumtemperatur gelöst. Diese Lösung tropft man anschließend innerhalb 45 min. in eine Lösung aus 2 ml 40 %ige Natronlauge, 2.9 ml Eisessig und 100 g Eis. Die Fällung der Kupplungskomponente wird auf 45°C erwärmt. Dann wird die über Aktivkohle geklärte Diazotierung innerhalb 30 min. zugetropft. Gleichzeitig wird 40 %ige Natronlauge so zugetropft, daß der pH-Wert der Mischung 4.0 - 4.5 beträgt. Die erhaltene Pigmentsuspension wird 1 Std. auf 80°C geheizt, abgesaugt und mit Wasser neutral gewaschen. Das gelbe Pigment der Formel

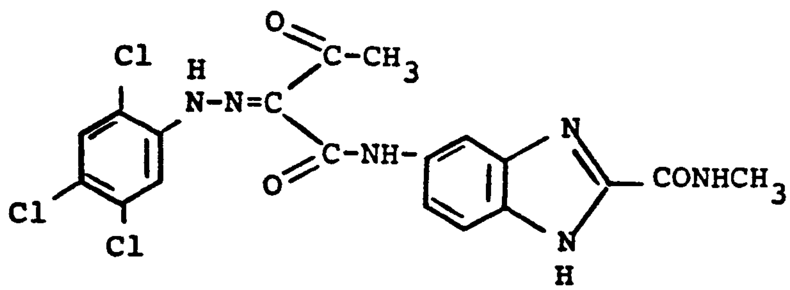

wird bei 70°C im Umlufttrockenschrank getrocknet.

Um ein möglichst deckendes und lichtechtes Pigment zu erhalten, wird es in Toluol 3 Std. auf 130°C erhitzt und anschließend auf übliche Weise isoliert.

Analog können nach bekannten Diazotier- und Kupplungsverfahren folgende Pigmente dargestellt werden:

| Bsp. | Diazokomponente | Kupplungskomponente | Nuance |
|---|---|---|---|
| 2 | 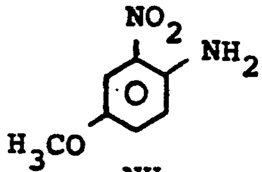 | 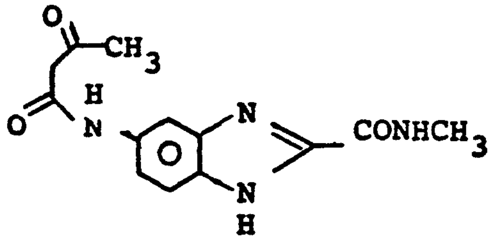 | gelbstichig-orange |
| 3 | 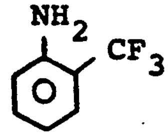 | " | grünstichig-gelb |
| 4 | 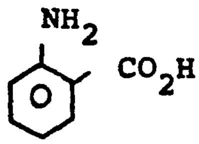 | " | gelb |
| 5 | 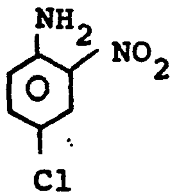 | " | gelb |
| 6 | 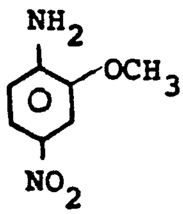 | " | gelb |
| 7 | 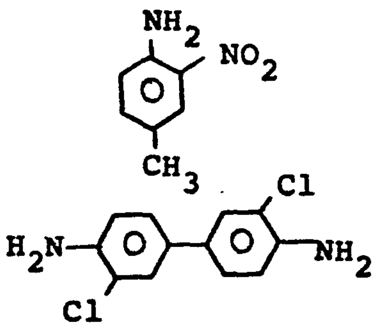 | " | gelb-braun |
| 8 | | " | rotstichig-gelb |
| 9 | 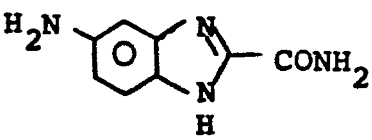 | " | gelb |
| 10 | 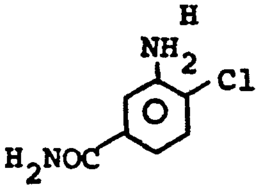 | " | gelb |



Le A 23 239

Beispiel 11

3.0 g 2,4,5-Trichloranilin werden nach Beispiel 1 diazotiert.
4.14 g der Verbindung

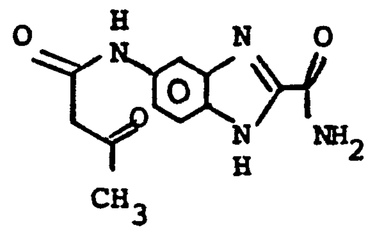

werden nach Beispiel 1 gefällt und auf 45°C aufgeheizt. Dann werden 20 ml Ethanol hinzugefügt. Anschließend wird die über Aktivkohle geklärte Diazotierung auf einmal zugegeben. Nach ca. 1 Std. ist die Kupplung beendet. Die erhaltene Pigmentsuspension wird 1 Std. auf 90°C geheizt, abgesaugt und mit Wasser neutral gewaschen. Das goldgelbe Pigment wird bei 70°C im Umlufttrockenschrank getrocknet.

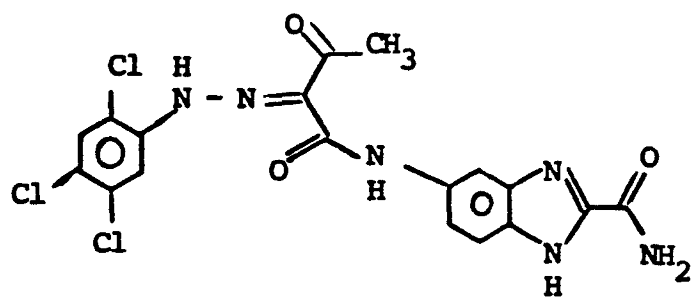

Um ein möglichst deckendes und lichtechtes Pigment zu erhalten, das auch kornweich ist, wird es in Butanol 1 Std. auf 150°C erhitzt und anschließend auf übliche

Weise isoliert.

Analog können nach im Prinzip bekannten Diazotier- und Kupplungsverfahren die folgenden Pigmente hergestellt werden:

| Bsp. | Diazokomponente | Kupplungskomponente | Nuance |
|---|---|---|---|
| 12 | 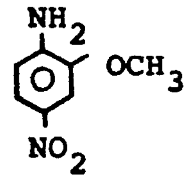 | 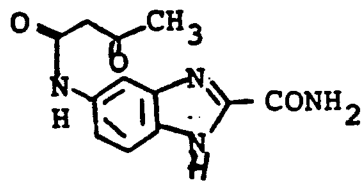 | gelb |
| 13 | 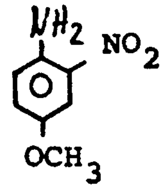 | " | rotstichig-orange |
| 14 | 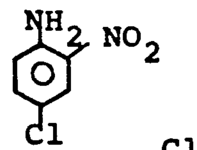 | " | rot-orange |
| 15 | 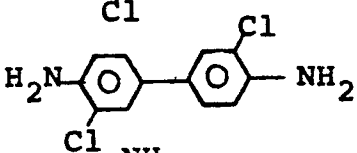 | " | rotstichig-gelb |
| 16 | 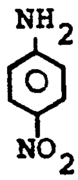 | " | grünstichig-gelb |
| 17 | 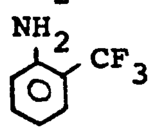 | " | gelb |
| 18 | 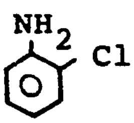 | " | grünstichig-gelb |
| 19 | 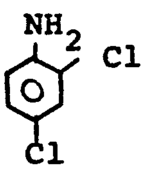 | " | gelb |
| 20 | 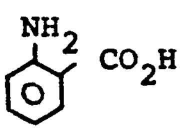 | " | grünstichig-gelb |

Beispiel 21

27.5 g 5-Amino-benzimidazol-2-carbonsäuremethylamid werden in 200 ml Wasser mit 40 ml Salzsäure (36 g HCl in 100 ml) gelöst. Durch Zugabe von Eis wird auf 0-5°C gekühlt. Innerhalb von 15 min. werden 34.5 ml 30 %ige Natriumnitrit-Lösung zugetropft. Es wird 1 Std. bei 0-5°C nachgerührt. Überschüssiges Nitrit wird dann durch Zugabe von Amidosulfonsäure zerstört.

43.28 g der Verbindung

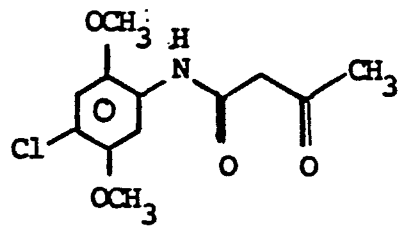

werden in 300 ml Wasser mit 20 ml 40%iger Natronlauge gelöst und über A-Kohle geklärt. Die Lösung wird innerhalb 45 min. zu einer Mischung aus 100 ml Wasser, 200 g Eis, 14 ml Eisessig und 100 ml 20 %ige Natriumacetat-Lösung getropft. Es werden 400 ml Ethanol zu gefällten Kupplungskomponente gegeben. Zu dieser Suspension wird innerhalb 1 Std. die über A-Kohle geklärte Lösung der Diazonium-Verbindung getropft. Die erhaltene Pigment-Suspension wird 1 Std. auf 80°C geheizt. Das gelbe Pigment wird bei 70°C im Umlufttrockenschrank getrocknet.

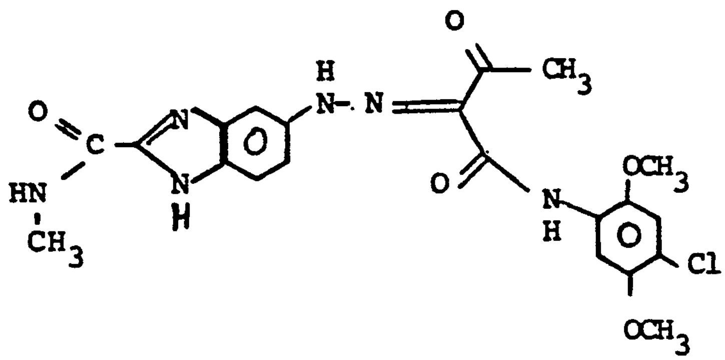

Um ein möglichst deckendes und lichtechtes Produkt zu erhalten wird es entweder in Butanol oder in einem Gemisch Butanol:Wasser = 1:1 (Volumenteile) 3 Std. auf 130°C erhitzt.

Analog können nach bekannten Kupplungsverfahren folgende Verbindungen dargestellt werden:

| Bsp. | Diazokomponente | Kupplungskomponente | Nuance |
|---|---|---|---|
| 22 | 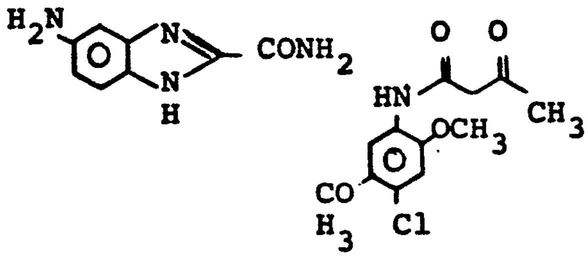 | | grünstichig-gelb |
| 23 | " | 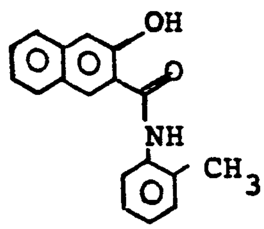 | bordeaux |
| 24 | " | 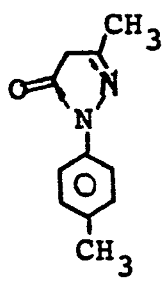 | gelb-orange |
| 25 | " | 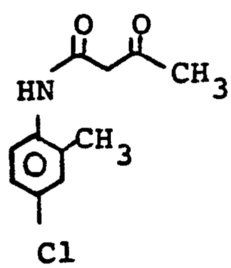 | gelb |
| 26 | " | 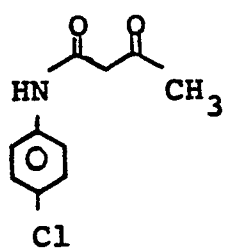 | gelb |
| 27 | " | 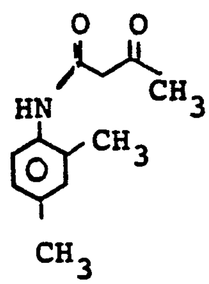 | gelb |

0170987



| Bsp. | Diazokomponente | Kupplungskomponente | Nuance |
|---|---|---|---|
| 28 | 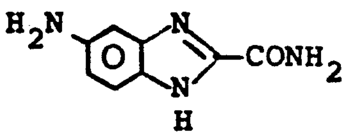 | 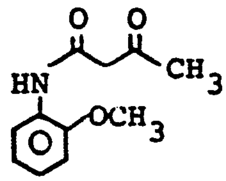 | grünstichig-gelb |
| 29 | " | 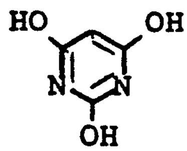 | gelbstichig-orange |
| 30 | 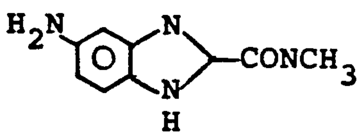 | 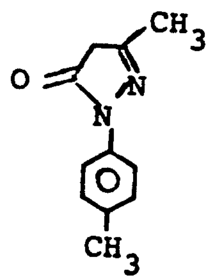 | rotstichig-orange |
| 31 | " | 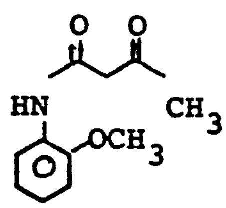 | gelb |
| 32 | " | 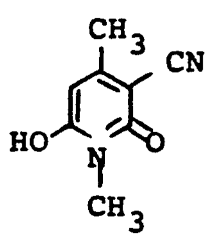 | gelb |

Beispiel 33

1.88 g 2-Hydroxy-3-naphthoesäure und 1.85 g 5-Amino-benzimidazol-2-carbonsäure-methylamid werden in 25 ml Toluol suspendiert. Bei 70°C werden innerhalb ca. 30 min. 1.2 ml Phosphortrichlorid zugetropft. Nach beendeter Zugabe wird 12 Std. unter Rückfluß erhitzt. Nach dem Abkühlen auf Raumtemperatur wird vorsichtig mit 50 ml Wasser versetzt und mit Soda auf pH 9 gebracht. Das Toluol wird durch Einleiten von Wasserdampf abdestilliert. Danach wird der Niederschlag abgesaugt und mit Wasser neutral gewaschen. Das erhaltene Produkt besteht aus:

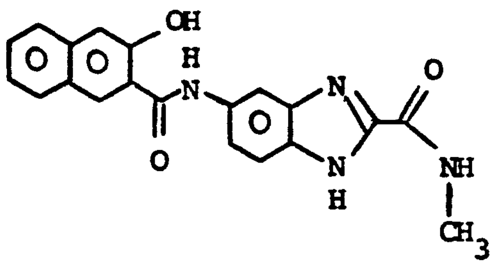

Nach im Prinzip bekannten Diazotier- und Kupplungsverfahren können die folgenden Pigmente hergestellt werden:

| Bsp. | Diazokomponente | Kupplungskomponente | Nuance |
|---|---|---|---|
| 34 | 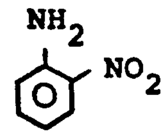 | 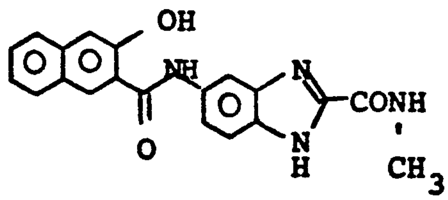 | blaustichig-rot |
| 35 | 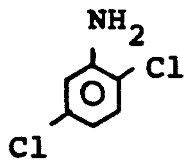 | " | karminrot |
| 36 | 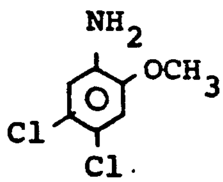 | " | marron |
| 37 | 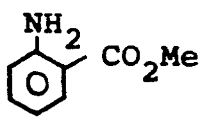 | " | rot |
| 38 | 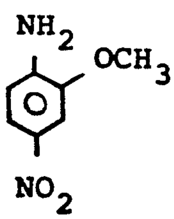 | " | blaustichig-rot |

Patentansprüche

1. Benzimidazol-2-carbonsäure-Derivate die in einer ihrer tautomeren Formen der Formel

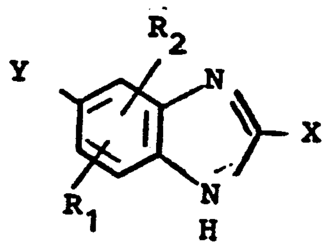

I

entsprechen, in der

$R_1$, $R_2$ H, Halogen, -R', -OR', -SR', -OH, $-\underset{\substack{\| \\ O}}{C}-R'$, $-O-\underset{\substack{\| \\ O}}{C}-R'$, $-NH-\underset{\substack{\| \\ O}}{C}-R'$, $-\underset{\substack{\| \\ O}}{C}-NH-R'$, $-\underset{\substack{\| \\ O}}{C}-N{<}^{R'}_{R''}$, $-\underset{\substack{\| \\ O}}{C}-NH_2$, $-SO_2-NH_2$, $-SO_2-NH-R'$, $-SO_2-N{<}^{R'}_{R''}$, $-NH-SO_2R'$, -CN, $-\underset{\substack{\| \\ O}}{C}-OH$ und $-NO_2$ bezeichnen, wobei

R', R" für gegebenenfalls substituiertes Alkyl, gegebenenfalls substituiertes Cycloalkyl, gegebenenfalls substituiertes Aryl und gegebenenfalls substituiertes Aralkyl stehen,

X eine Carboxylfunktion oder derivatisierte Carboxylfunktion bezeichnet und

Y für einen Rest der Formeln

a) $-NH_2$

b) 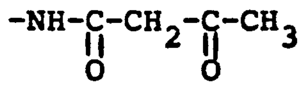

c) 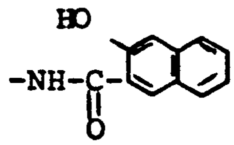

d) 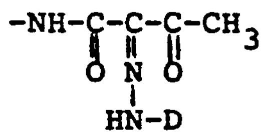

e) 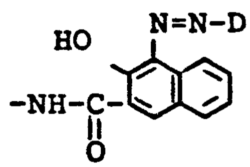

f) -N=N-K steht,

wobei

D den Rest einer Diazokomponente und

K den Rest einer Kupplungskomponente bezeichnen.

2. Benzimidazol-2-carbonsäurederivate gemäß Anspruch 1, bei denen Halogen Cl, F und Br bezeichnet; gegebenenfalls substituiertes Alkyl $C_1$-$C_8$-Alkyl, das durch $C_1$-$C_4$-Alkoxy, $C_1$-$C_4$-Alkylthio, Halogen, Hydroxy, Cyan, Nitro, Amino, Mono- und Di-$C_1$-$C_4$-alkylamino, Carboxy,

$C_1$-$C_4$-Alkoxy-carbonyl, Sulfo, $C_1$-$C_4$-Alkylsulfonyl und Arylsulfonyl mit 6 bis 10 C-Atomen im Arylteil substituiert sein kann, bezeichnet; gegebenenfalls substituiertes Cycloalkyl mono-, bi- und tricyclisches Cycloalkyl mit 3 bis 10 C-Atomen, wobei die Cycloalkylreste durch $C_1$-$C_4$-Alkoxy, $C_1$-$C_4$-Alkylthio, Halogen, Hydroxy, Cyan, Nitro, Amino, Mono- und Di-$C_1$-$C_4$-alkylamino, Carboxy, $C_1$-$C_4$-Alkoxy-carbonyl, Sulfo, $C_1$-$C_4$-Alkylsulfonyl und Arylsulfonyl mit 6 bis 10 C-Atomen im Arylteil substituiert sein können, bezeichnet; gegebenenfalls substituiertes Aryl Aryl mit 6 bis 10 C-Atomen im Arylteil, wobei die Arylreste durch $C_1$-$C_4$-Alkyl, $C_1$-$C_4$-Alkoxy, $C_1$-$C_4$-Alkylthio, Halogen, Hydroxy, Cyan, Nitro, Amino, Mono- und Di-$C_1$-$C_4$-alkylamino, Carboxy, $C_1$-$C_4$-Alkoxy-carbonyl, Sulfo, $C_1$-$C_4$-Alkylsulfonyl und Arylsulfonyl mit 6 bis 10 C-Atomen im Arylteil substituiert sein können, bezeichnet; gegebenenfalls substituiertes Aralkyl Aryl-$C_1$-$C_6$-alkyl, wobei der Arylrest durch $C_1$-$C_4$-Alkyl, $C_1$-$C_4$-Alkoxy, $C_1$-$C_4$-Alkylthio, Halogen, Hydroxy, Cyan, Nitro, Amino, Mono- und Di-$C_1$-$C_4$-alkylamino, Carboxy, $C_1$-$C_4$-Alkoxy-carbonyl, Sulfo, $C_1$-$C_4$-Alkylsulfonyl und Arylsulfonyl mit 6 bis 10 C-Atomen im Arylteil substituiert sein kann, bezeichnet.

3. Benzimidazol-2-carbonsäure-Derivate gemäß den Ansprüchen 1 - 2, bei denen $R_1$ und $R_2$ für Wasserstoff stehen.

4. Benzimidazol-2-carbonsäure-Derivate gemäß den Ansprüchen 1 - 3, bei denen X für -COOH, -COOR', -CONHR', 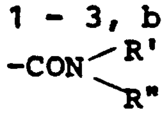 und -CN steht.

5. Benzimidazol-2-carbonsäure-Derivate gemäß den Ansprüchen 1 - 3, bei denen X für -COOH, $-COOCH_3$, $-COOC_2H_5$, $-CONH_2$, $-CONHCH_3$, $-CONHC_2H_5$, $-CON(CH_3)_2$, $-CON(C_2H_5)_2$ und 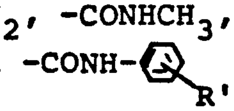 steht.

6. Benzimidazol-2-carbonsäure-Derivate gemäß den Ansprüchen 1 - 5, bei denen D für den Rest einer Diazokomponente der Benzol-, Naphthalin-, Anthrachinon- oder heterocyclischen Reihe steht.

7. Benzimidazol-2-carbonsäure-Derivate gemäß den Ansprüchen 1 - 5, bei denen D einen Phenyl-, Naphthyl- oder Anthrachinonyl-Rest, die 1,2 oder 3 Substituenten $R_1$ tragen können, einen gegebenenfalls substituierten Barbitursäurerest oder einen Rest der in einer seiner tautomeren Formen der Formel

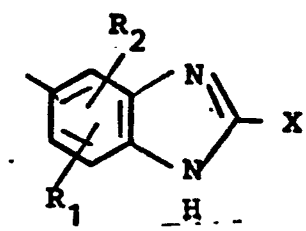

II

entspricht,

bezeichnet.

8. Benzimidazol-2-carbonsäure-Derivate gemäß den Ansprüchen 1 - 7, bei denen K eine Kupplungskomponente aus der Benzol-, Naphthalin-, Acetessigamid- oder heterocyclischen Reihe bezeichnet.

9. Benzimidazol-2-carbonsäure-Derivate gemäß den Ansprüchen 1 - 7, bei denen K einen Rest, der sich von einem 2-Hydroxy-3-napthoesäurearylid, einem Acetessigarylid, einer gegebenenfalls substituierten Barbitursäure, einem Pyrazolon oder einem 3-Cyan-4-methyl-2,6-pyridon ableitet, wobei unter Arylid ein Phenylrest verstanden wird, der durch $R_1$ substituiert sein kann oder an den ein heterocyclischer Rest ankondensiert sein kann, bezeichnet.

10. Verwendung von Verbindungen der Formeln Id, Ie und If gemäß den Ansprüchen 1 - 8 als Pigmente.

Europäisches Patentamt

# EUROPÄISCHER RECHERCHENBERICHT

0170987

Nummer der Anmeldung

EP 85109288.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 98, Nr. 17, 25. April 1983, Columbus, Ohio, USA<br>P.A. PETYUNIN et al. "Synthesis and properties of N-R-amides of benzimidazole-2-carboxylic acid" Seite 571, Spalte 2, Zusammenfassung Nr. 143 322r<br>& Farm. Zh. (Kiev) 1982, (6), 59-61.<br>---- | 1 | C 07 D 235/24<br>C 07 D 401/12<br>C 07 D 403/12<br>C 09 B 29/036 |

| RECHERCHIERTE SACHGEBIETE (Int. Cl.4) |
|---|
| C 07 D 235/00<br>C 07 D 401/00<br>C 09 B 29/00<br>C 09 B 57/00<br>C 09 B 51/00<br>C 09 B 43/00 |

Der vorliegende Recherchenbericht wurde für alle Patentansprüche erstellt.

| Recherchenort | Abschlußdatum der Recherche | Prüfer |
|---|---|---|
| WIEN | 06-11-1985 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82